# EUROPEAN PATENT SPECIFICATION

(11) **EP 1 448 071 B1**
(45) Date of publication and mention of the grant of the patent: **12.01.2011**
(21) Application number: 02757446.6
(22) Date of filing: 29.08.2002
(51) Int. Cl.: A41D 19/00, A61B 17/06, B65D 85/18

(54) **SURGICAL DOUBLE GLOVE SET AND PACKAGING THEREFOR**
CHIRURGISCHES DOPPELTES HANDSCHUHSET UND VERPACKUNG DAFÜR
DOUBLE PAIRE DE GANTS CHIRURGICAUX ET SYSTÈME D'EMBALLAGE CORRESPONDANT

(30) Priority: 29.08.2001 US 315314 P
(43) Date of publication of application: 25.08.2004
(73) Proprietor: Low, Chin Guan, Lutz, FL 33549-9703 (US)
(72) Inventor: Low, Chin Guan, Lutz, FL 33549-9703 (US)
(74) Representative: Hedley, Nicholas James Matthew
(86) International application number: PCT/US2002/027442
(87) International publication number: WO 2003/020109

(56) References cited:
- US-A- 3 412 851
- US-A- 3 412 851
- US-A- 4 094 120
- US-A- 4 545 841
- US-A- 5 636 382

## Description

### BACKGROUND OF THE INVENTION

### 1. Field of the Invention

The present invention is directed to a double glove set for use in medical applications, and more particularly in surgical applications, and to a system and method for packaging the double glove set.

### 2. Description of Related Art

Orthopedic surgery and/or trauma surgery is a higher risk procedure in comparison to general surgery. The nature of the work involves the use of power tools and other equipment that necessitates placing the surgeons' hands in close proximity to surgical tools and cutting bits. Such tools and cutting bits cause approximately 10 - 35 % of glove tears during procedures, according to the Medical Device Association. Some tears go unnoticed until after the surgical procedure. In trauma operations, treating physical injury resulting from force, there is also high use of equipment which is frequently used without providing advance notice to the doctor. This is due to the immediate attention needed for the patient and the shortness of time for preoperation testing, and this situation puts doctors at a disadvantage as far as cross-infection risks are concerned.

In view of the high risk of glove tears and the prevalence of HIV and Hepatitis infection risk, many surgeons are looking for better protection in the form of thicker gloves and even wearing two pairs of gloves. These solutions raise the issues of sensitivity and comfort for the surgeons who commonly work 8-10 hours per operation. In such long procedures, the stress on the hands of the surgeons can be numbing and in many cases cramps develop when two pairs of gloves are used. This cramping is caused by excessive pressure being exerted on the hands and fingers by the double gloves, and also on account of the extra strength needed to move the fingers in two sets of gloves.

The use of heavier gauge gloves does not give better protection, and works to impede the dexterity required of the person wearing the gloves. The concept of double gloving is supposed to provide increased protection to the surgeons, specialists, etc., without diminishing the dexterity of the wearer. Currently, if a person wishes to wear double gloves, the present choice is to wear two pairs of the same size glove, or of different sized gloves, or of different models. Any of these create a logistics problem, and/or discomfort from wearing two pairs of the same size or different sizes. Surgeries further require that the surgeon wear a well fitted glove without any slack or excess loose material being present.

Existing products in market are for single gloving. Each pair is packed into a sterile pack. Anyone who wishes to wear two pairs at a time has two options:
(a) Wear two pairs of similar sized product; or
(b) Wear one size bigger for the outer pair.

Both options require the surgical room team to prepare, on the operating tables, two pairs of gloves for each change in gloves. A surgeon can go through 3- 6 changes in an operation. Then there is also the problem of matching the inner and outer pairs as per options (a) and (b) listed above.

It is a principal object of this invention to eliminate or reduce significantly the above-noted disadvantages, and to provide surgeons with more comprehensive protection and comfort.

It is a further principal object of the invention to provide gloves for orthopedic surgeons, trauma specialists, healthcare personnel etc. , that may be made from natural or synthetic elastomeric materials, e.g., natural latex and synthetic latices of nitrile, polychloroprene polyurethanes and tactylon material, or mixtures of the mentioned materials.

It is an additional principal object of the present invention to provide a packaging arrangement for the double gloves to improve the logistics or supply issues, and to facilitate the donning of the gloves by surgeons or other healthcare personnel.

US 5,636,382 describes a protective glove system to prevent the skin from unwanted exposure to contaminants and finds application in the household and work environment, particularly in healthcare facilities. The glove system has an inner glove and an outer glove. The inner glove maybe worn continuously while the outer glove may be changed. The glove system should be snug but not constricting and should provide confident tactility. The two-part form of claim 1 is based on this document.

US 3,412,851 describes a method of wrapping a single pair of surgical gloves by enclosing the gloves within a folded single wrapper sheet. The sheet is provided with tabs to open the wrapper.

### SUMMARY OF THE INVENTION

The above and other objects of the present invention are obtained by providing a double glove arrangement in which the inner and outer gloves may be sized to provide long-lasting comfort, and may be of a composition which produces desired material properties contributing to the comfort in extended wear of the gloves.

The present invention is embodied in a packaged double surgical glove set, comprising: a first pair of inner gloves; a second pair of outer gloves to be worn over said inner gloves; said first pair and said second pair of gloves being separated by a separator sheet, with said first pair of gloves overlying said second pair of gloves; said first pair and said second pair of gloves and said separator sheet being packaged in a single pack.

The two pairs of gloves may be orthopedic surgeon's gloves that are hand specificand comprise:
a) a pair of inner primary surgeon's gloves, preferably bright yellow or apple green in color, and
b) a pair of outer secondary gloves, preferably tan in color, that are not fixed, glued or bonded to the inner glove, allowing users to independently peel off each pair or each glove.

According to the present invention, a surgeon need only open a single pack of the desired size and there will be two pairs of gloves present to wear. The first pair, which will be referred to at times as the primary glove pair, is preferably colored bright yellow or apple green is presented and will be donned first. Then the second pair, referred to at times as the secondary glove pair, is preferably tan in color, is presented and may be donned by the wearer. The color difference allows for a clear distinction between body color and glove as compared to conventional brown orthopedic gloves or natural latex colored gloves in the market.

In the double surgical glove set:
- the second pair of outer glovesmay be about 3% to about 4.5% larger than said gloves of said first pair,
- the thickness of finger tip and palm areas of said gloves of said first pair may be in a range of about 0.14 mm to about 0.20 mm, the thickness of a finger tip area of said gloves of said second pair may be in a range of about 0.24 mm to about 0.30 mm, and the thickness of a palm area of said gloves of said second pair may be in a range of about 0.21 mm to about 0.27 mm.

The double set of surgical gloves may be donned using the following method:
(a)opening the packaged glove set according to the present invention to access said first and second pair of gloves;
(c) donning said first pair of gloves; and
(d) donning said second pair of gloves.

The glove set according to the present invention may be packaged by:
(a) providing an outer sterile field sheet ;
(b) positioning a pair of hand-specific outer gloves in a side-by-side orientation on said outer sterile field sheet;
(c) positioning a separator sheet atop said pair of hand-specific outer gloves;
(d) positioning a pair of hand-specific inner gloves in a side-by-side orientation atop said separator sheet; and
(e) then sealing the thus assembled outer sterile field sheet, the pair of outer gloves, the separator sheet, and the pair of inner gloves inside a single sterile pack.

### BRIEF DESCRIPTION OF THE DRAWINGS

The above and other features of the invention and the attendant advantages will be more fully appreciated by having reference to the following detailed description of the presently preferred embodiments, taken in conjunction with the accompanying drawings, in which:
FIG. 1 is a substantially schematic view of the layout of a primary and a secondary pairs of gloves not in accordance with the present invention
FIG. 2 is a perspective view of the placement of the pairs of gloves into the packaging according to a preferred embodiment of the present invention.
FIG. 3 is a substantially schematic view of a glove folded for packaging in accordance with the prior art.
FIG. 4 is a substantially schematic view of a glove folded for packaging in accordance with a preferred embodiment of the present invention.
FIG. 5 is a substantially schematic view of the pairs of gloves packaged inside a sterile pack in accordance with a preferred embodiment of the present invention.

### DETAILED DESCRIPTION OF THE PREFERRED EMBODIMENTS

Orthopedic surgical gloves currently on the market are a thicker version of general surgery gloves, are brown in color, and are packed with a single pair per pack. The thickness of conventional orthopedic gloves varies from 0.25 mm to 0.30 mm at the palm to 0.30 mm to 0.35 mm at the fingertips. The normal modulus at 500% (stress of stretching to a certain percentage from rest, usually measured at units of MPa at 300% or units of MPa at 500% of stretch) is in the 4.0 MPa to 4.5 MPa range. ASTM Standard D412 is a widely accepted standard for determining these physical properties.

Wearing two of this type of conventional glove on each hand will exert a significant amount of pressure on the hands of the surgeon, causing the hands or fingers to cramp, or both, after long procedures, which are typical of orthopedic operations.

The present invention provides a double glove set in which:
1. an inner pair of gloves is specially formulated and manufactured to be:
   a. thin (preferably about 50 % of the usual thickness of conventional orthopedic gloves),
   b. highly stretchable (preferably about 960% or more elongation at break, compared to 800 % elongation at break for existing gloves), and
   c. Low modulus (2.5 - 3.5 MPa @ 500%, compared to 4.0-4.5 MPa @500% for existing gloves); and
2. an outer pair of gloves is of a thicker gauge than the inner pair and
   a. preferably is about 85-90% of thickness of typical orthopedic gloves, and
   b. has a low modulus (preferably about 900% or more elongation at break compared to 800 % elongation at break for conventional gloves), with the following properties:
      I. high stretchable properties (920% or more elongation at break compared to 800 % elongation at break for conventional existing gloves); and/or
      II. low modulus (2.5 - 3.5 MPa @ 500%, compared to 4.0-4.5 MPa @500% for conventional existing products) Table 1 below presents an example of the preferred physical properties of latex gloves provided in accordance with the present invention.

**Table 1**

| **Measured according ASTM D 412** | **Inner Primary Preferred range** | **Outer Secondary Preferred range** |
|---|---|---|
| Elongation at break | 800 - 960% | 800-900 % |
| Modulus at 500% | 2.5 - 3.5 MPa | 2.5 - 3.5 MPa |

The above properties may be achieved through the use of the compounding formula set forth in Table 2, for natural latex gloves. The percentages shown are by weight.

**Table 2**

| Chemical | Inner Primary **Preferred range** | Outer Primary **Preferred range** |
|---|---|---|
| Sulphur | 0.5 - 0.9 % | 0.6 - 1.0 % |
| Zinc Oxide | 0.3 - 0.8 % | 0.4 - 0.8 % |
| Zinc diethyl dithiocarbamate (ZDEC) and Zinc dibuthyl dithiocarbamate (ZDBC) | 0.65 - 1.2 % | 0.65 - 1.2 % |
| Antioxidant Sterically hindered phenol Butylated p-cresol and dicyclopentadiene | 0.8 - 1.6 % | 0.8 - 1.6 % |
| Plasticizer Oil | 0.3 - 0.8% | 0.3 - 0.8% |

The lower sulphur content allows for a higher elongation or stretching ability. A preferred range of sulphur in the latex formulation is 0.5 - 0.9 % by weight for the inner primary pair of gloves. In general, it is preferred that the sulphur content be kept low when the level of non-latex impurities in the latex compound is higher.

Zinc oxide acts as an activator to the sulphur and is preferably present in the range of 0.3 - 0.7 % by weight. This chemical will also create a harder end product and also acts as a destabilizer to the latex, hence its activator role. Latex on its own is rather stable. To create rubber products, the liquid form of the rubber must be destabilized to coagulated into rubber. This is assisted by dipping into calcium nitrate or calcium chloride, the calcium ions of which will coagulate the latex into blocks or films. A higher percentage than the preferred values will create a stiffer glove having a modulus of more than 4.4 MPa at 500%. This means more strength is required to stretch the product to the same elongation. As noted previously, this results in excessive pressure exerted on the hands of the wearer. It is therefore an important factor to achieve the preferred modulus.

The addition of ZDEC (zinc diethyl dithiocarbamate) and/or ZDBC (zinc dibuthyl dithiocarbamate) is to accelerate the cross linking of the rubber molecules with the bond supplied by the sulphur molecules. This determines the amount of cross linking of sulphur -sulphur bonds and the rate at which it is done. It also has a bearing on the modulus of the final film created. Percentages higher than those set forth in Table 2 will generally create a weaker glove with lower sterilization aging resistance, and also will tend to cause the ZDEC and ZDBC to bloom to the surface of the latex.

An antioxidant added to the compound will provide protection against oxidative aging and also irradiation aging during sterilization. The range set forth in Table 2 is believed to be the optimum for a thin walled product like the gloves of this invention.

The plasticizer added is preferably in the form of parafinic oil. The parafinic oil is preferred to be light in color, so that it does not discolor the product. The percentage of plasticizer oil for the inner primary glove is preferably 0.3- 0.8% by weight, which helps to ensure that the product has a high elongation and softness, which also contributes to achieving the modulus in the preferred range. Excess parafinic oil (substantially in excess of the Table 2 range) will bloom to the surface, reducing the effectiveness of the additive.

The gloves of the present invention may be produced in two versions, powdered and powder free. To aid a wearer in donning the gloves, approved powdered absorbable cornstarch is used. This allows smooth insertion of hands into the gloves.

The powder free version of the gloves will preferably employ a second material added to the gloves just before they leave the production line. The gloves, together with the molds for manufacturing the gloves, are dipped in a water based polyacrylic emulsion, creating a microthin layer of acrylic on the inside surface of the glove (the glove is inverted when removed from the mold) that will assist in donning the gloves. This acrylic-coated glove is then halogenated with soluble chlorine, removing the powder from the glove, and at the same time smoothing the natural surface of the latex glove, which is tacky in its original form.

As noted previously, the double gloving as practiced in the past required the wearer to don two pairs of gloves of the same size, or to use an outer pair of the next larger size. This creates two problems. With the first option, the hands of the surgeons are tightly squeezed by the outer gloves. To understand this, it will be recognized that surgeons always wear their gloves tight or hugging tight, so that no loose portions of the gloves, such as wrinkles or creases, or sagging material, are present. This enables the precise fine movements that a surgeon requires. Wearing two pairs of the same size, however, creates excess pressure on the hands from the outer pair.

Wearing a larger size for the outer gloves gives rise to slackness in the outer gloves and folds or loose parts are present. The larger outer glove will not closely conform to the inner glove, nor to the hand. This is due at least in part to the fact that it is international standard that the next size up for each larger pair of gloves is 6-10% wider than the next smaller glove in all dimensions. The lack of close conformity is highly undesirable, and, in may cases, unacceptable, for performing orthopedic surgery.

In order to avoid these problems the present invention provides:
- a thin inner primary glove
- a dimensionally larger outer secondary glove
- a longer outer secondary glove
- a bright color contrasting alert system

The inner primary glove 100 is a glove of standard international size (as per ASTM 3577-2001 definition). This is the size that is understood by the surgeons and which they presently use. The pair of inner primary gloves of the present invention are made 30-50% thinner than the standard orthopedic surgical glove. A preferred range of thicknesses of the glove material is presented in Table 3 below. In addition to having a lower thickness, the gloves have a lower modulus, as discussed earlier. This creates a glove 100 that is almost like a second skin, fitting well over the surgeon's hands and exerting three times less pressure than a normal glove.

The outer secondary glove 102 is 10 -15% thinner than existing orthopedic gloves, is lower in modulus and is dimensionally only 3 % - 4.5% larger than the inner primary glove 100, which means that it can be worn over the inner primary glove 100 and not exert the high level of extra pressure on the hands as would be the case if both gloves are of the same size. Furthermore, this small increase in size over the inner glove size is not as large as employing the next larger standard size glove, which, as noted previously, is 6-10% wider. As such, the outer glove fits well and hugs the contours of the inner glove and the hands and fingers of the wearer, with minimal pressure. It is to be noted that the above percentages cited are size dependent.

The thicknesses of the two gloves in this double glove set 10 are selected with the understanding that one does not actually need the two gloves to be of equal thickness so as to provide double protection, but rather the inner glove is to provide a second effective barrier that protects the user temporarily, should the outer glove fail. Should the outer glove fail, the wearer will need to be aware of such a fact and will have been instructed to change gloves immediately, rather than relying on just the inner glove. The inner glove, however, provides the necessary and desirable interim protection.

The cuff portion 104 of the outer secondary glove is equal to, or, preferably, longer than, the cuff portion of the inner glove. Two pairs of equal length gloves can sometimes present problems with both cuffs ending at the same position, whereby the outer gloves are prone to slipping back over the smooth surface of the inner gloves, causing a gathering or bunching of material along the forearm and/or near the wrist. Employing a longer outer glove 102 will allow the longer glove to separately engage the clothing or skin of the wearer at a point further up the arm from where the inner glove stops. This essentially eliminates the glove-over-glove slippage that can occur when the outer glove 102 engages only the outer surface of the inner glove 100.

Because the inner glove is significantly thinner than a standard orthopedic glove, the protection it provides in the event of an outer glove failure is best regarded as being for interim protection only. Accordingly, the set of gloves preferably provides an alert mechanism in which the color of the inner primary glove is made bright yellow or apple green that will give a sharp contrast to body fluids containing blood which may penetrate the outer glove. This is the color contrast alert system in these gloves. No chemicals are added to the surface of the gloves or to the composition of the gloves to produce color changes in reaction to contact with body fluid, an approach that has previously been used. The original bright contrasting color of the inner glove is sufficient. When the wearer notes the presence of the sharp contrast between body fluids and the inner glove, the wearer is alerted that a glove change should be made as soon as is possible.

The following tables state preferred ranges of various dimensions, etc., of the gloves 100, 102 according to the present invention.

**Table 3 - Thickness**

| Measured as per ASTM D 3577 | Inner Primary | Outer Secondary |
|---|---|---|
| Fingertips | 0.14 mm to 0.20 mm | 0.24 mm to 0.30 mm |
| Palm | 0.14 mm to 0.20 mm | 0.21 mm to 0.27mm |
| Cuff | 0.13 mm to 0.19 mm | 0.17 mm to 0.23 mm |

**Table 4 - Length**

| Measured as per ASTM D 3577 | Inner Primary | Outer Secondary |
|---|---|---|
| Index finger tips to cuff end | 280 - 290 mm | 295 mm - 310 mm |

**Table 5 - Color**

| | Inner Primary | Outer Secondary |
|---|---|---|
| Color | Bright Yellow/Apple Green | Tan |

**Table 6 - Dimensions: Size 6.5 (width measured in mm)**

| | ASTM D 3577 | Primary Glove | Secondary Glove |
|---|---|---|---|
| Index Finger | | 23 | 24-25 |
| Palm | 83 | 83 | 85-87 |
| Wrist | | 69 | 70-71 |

**Table 7 - Dimensions: Size 7 (width measured in mm)**

| | ASTM D 3577 | Primary Glove | Secondary Glove |
|---|---|---|---|
| Index Finger | | 26 | 27-28 |
| Palm | 89 | 89 | 90-92 |
| Wrist | | 80 | 81-82 |

**Table 8 - Dimensions: Size 7.5 (width measured in mm)**

| | ASTM D 3577 | Primary Glove | Secondary Glove |
|---|---|---|---|
| Index Finger | | 28 | 29-30 |
| Palm | 95 | 95 | 97-99 |
| Wrist | | 75 | 76-77 |

**Table 9 - Dimensions: Size 8.0 (width measured in mm)**

| | ASTM D 3577 | Primary Glove | Secondary Gove |
|---|---|---|---|
| Index Finger | | 30 | 31-32 |
| Palm | 102 | 102 | 104-106 |
| Wrist | | 87 | 88-89 |

In a further aspect of the present invention, the packaging of the double set of gloves is highly advantageous from a logistics and ease of use standpoint. Existing orthopedic gloves are packed in a pair per single sterile pack. In order to use two pairs for double gloving, one needs to open two packs. With several glove changes per operation and with more than one surgeon operating or assisting, there arises a problem of having too many packs of various sizes that need to be arranged on the operating room tables.

The present invention provides for double gloving needs with the use of only one single pack and thus reduces, by half, the quantity of material that needs to be arranged on operating room tables and further cuts down the volume of waste paper. This has the additional benefit of also reducing the cost of the product.

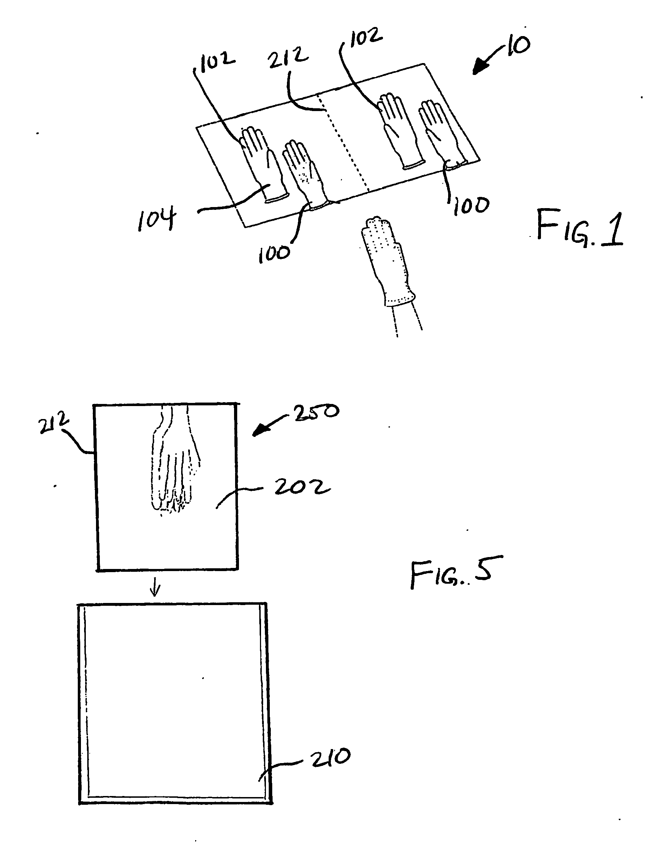

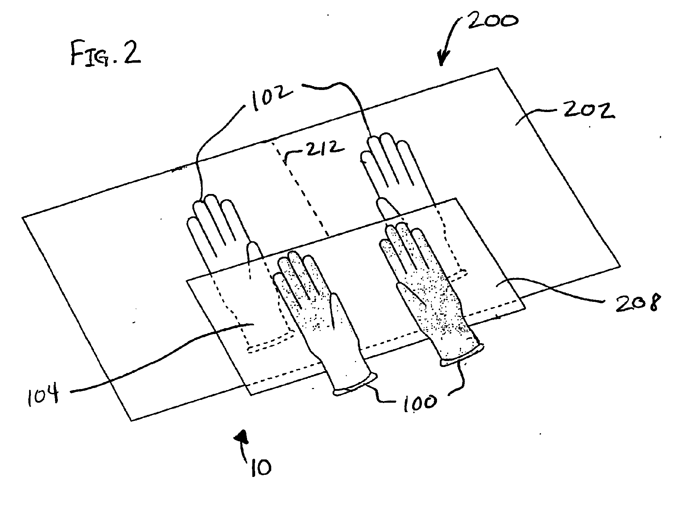

Figure 1 is not in accordance with the present invention but only because the glove set does not include a separator sheet 208, which is described in connection with Figure 2.

As shown in Fig. 2, the present invention provides a glove packaging system 200 that includes a single sheet of sterile field paper 202 that is used to wrap two pairs of gloves 100,102, which are hand specific. The two pairs of gloves are separated by a separator sheet 208. This is then sealed in a sterile packaging pack 210 (FIG. 5) for irradiation with gamma rays. Thus one pack will meet the complete needs of a surgeon to double glove.

In one exemplary embodiment, the sterile field paper 202 type is bleached Kraft coated paper weighing 40-50 grams per square meter. Other paper approved for medical packaging may also be used. Also in an exemplary embodiment, the separator sheet 208 is made from bleached Kraft coated paper weighing 40-50 gram per square meter. Other paper or synthetic film approved for medical packaging may also be used.

A preferred procedure for packing gloves according to the present invention involves spreading open the sterile field paper 202 and placing on it a pair of hand specific secondary gloves 102, palm up, and folded in a manner that will be described subsequently. The separator field or sheet 208 (40 - 50 gsm bleached Kraft paper) is placed over the secondary gloves 102, and the yellow colored or apple green colored primary gloves 100 are placed over the separator. The large sterile field 202 is folded at 212 over the two pairs in a conventional manner to create a folded pack 250 (FIG. 5). The folded pack 250 is then sealed inside a sterile packaging pouch for sterilization with gamma irradiation or ethylene oxide gas.

The separator sheet is generally necessary to stop migration of colors from the colored primary gloves to the secondary gloves adjacent thereto. This also serves to physically separate the gloves, which can be advantageous in the case where the gloves are a powderfree version, as this type is more tacky and tends to slightly stick together after compressed storage, creating a delay for the surgeon in separating the primary from the secondary gloves.

A further aspect of the present invention as it relates to the glove packaging system 200 is that the system provides an improved manner of folding the individual gloves 100, 102 within the folded pack 250 and field paper 202.

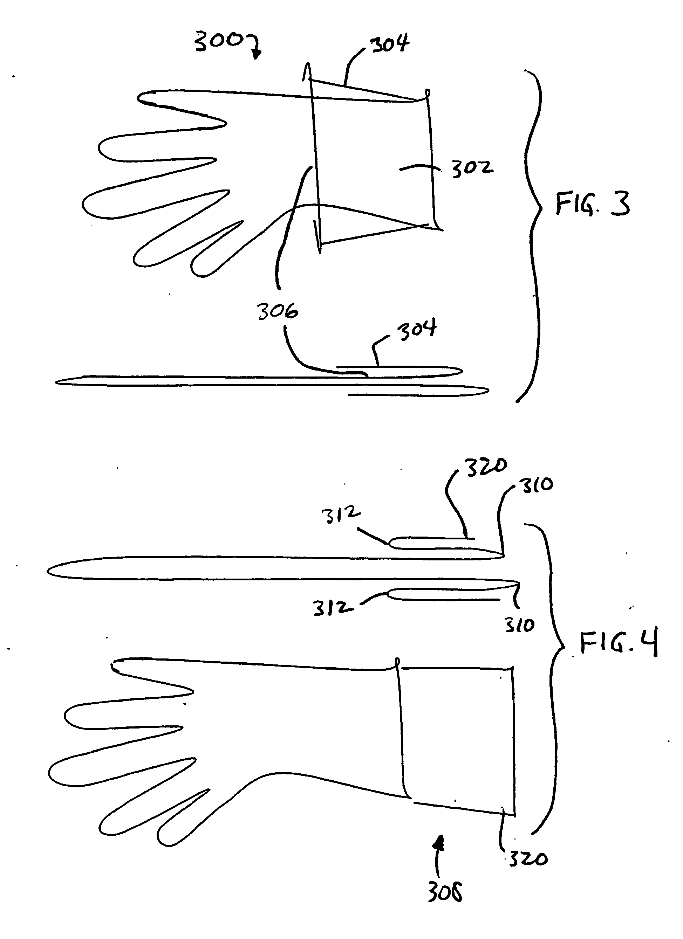

FIGS. 3 and 4 show, respectively, the folding of an orthopedic surgical glove 300 in accordance with the conventional practice, and in accordance with the present invention. The cuffs 302 of the gloves, as seen in FIG. 3, have conventionally been folded a single time, such that the distal end 304 of the cuff is doubled back over the proximal end 306, to the wrist area adjacent the palm and finger areas of the glove 300. This folding back of the cuff 302 eases the insertion of a hand into the palm and finger portion of the glove.

A drawback associated with the doubling over of the distal end 304 of cuff 302 was recognized in developing the present invention. When the distal end of the cuff is doubled over the proximal end, the distal end 304 is directly adjacent to or overlies a portion of the palm and/or finger area of the glove 300. When the surgeon proceeds to don the glove, the surgeon's bare hand is susceptible of contacting and contaminating the palm area of the glove.

FIG. 4 illustrates a packaging embodiment which substantially reduces the risk of contaminating the outer surfaces of the glove while donning the same. In this embodiment, a double fold, producing three overlying portions of the cuff 308, is provided. A fold 310 turning the cuff in the direction of the palm/finger portions of the glove is first made, and then a second fold 312 of the cuff, turning back the distal end 314 of the cuff in the direction away from the palm/fingers, is provided.

As a result of these two folds, the cuff portion 308 of the glove is positioned out of the way of the person's hand in donning the glove. However, the wearer will not be nearly as likely to contact and contaminate the critical palm and finger portions of the glove, in that he/she will grip the cuff 308 at a location that is remote from the palm section when the glove is put on. The wearer will either grip the very edge of the cuff 320, or will pull the glove on from fold 312. Either way, the gripping hand is considerably further away from the palm/finger portion of the glove, thereby decreasing the risk of contaminating the glove during surgery preparation.

While this invention has been described in conjunction with the specific embodiments outlined above, it is evident that many alternatives, modifications and variations will be apparent to those skilled in the art. Accordingly, the preferred embodiments of the invention, as set forth above, are intended to be illustrative, not limiting. Various changes may be made without departing from the scope of this invention as defined by the appended claims.

## Claims

1. A surgiral glove set, comprising:
a first pair of inner gloves (100,300); and
a second pair of outer gloves (102,300) to be worn over said inner gloves;
**characterised in that:**
the glove set is packaged as a double surgical glove set;
said first pair and said second pair of gloves are separated by a separator sheet (208), with said first pair of gloves overlying said second pair of gloves;
said first pair and said second pair of gloves and said separator sheet are packaged in a single pack.

2. A surgical glove set as set forth in Claim 1, wherein said first pair of inner gloves (100,300) and said second pair of outer gloves (102,300) each comprises two hand specific gloves.

3. A surgical glove set as set forth in Claim 2, wherein said gloves of said second pair of outer gloves (102,300) are positioned side-by-side on an outer sterile field sheet (202), and said gloves of said first pair of inner gloves (100,300) are positioned side-by-side overlying said second pair of outer gloves, with said separator sheet (208) disposed between said first pair and said second pair of gloves.

4. A surgical glove set as set forth in Claim 3, further comprising a sterile packaging pouch (210), wherein said outer sterile field sheet (202) is folded along a line extending between each pair of side-by-side inner and outer gloves (102,300) , said outer sterile field sheet having said first and said second pair of gloves disposed between folded-over portions of the folded sheet, and wherein said outer sterile field sheet and said first and said second pair of gloves are sealed within said sterile packaging pouch.

5. A surgical glove set as set forth in Claim 1, wherein said gloves of said second pair of outer gloves (102,300) are larger than said gloves of said first pair of inner gloves (100,300), e.g. said gloves of said second pair of outer gloves are about 3% to about 4.5% larger than said gloves of said pair of inner gloves.

6. A surgical glove set as set forth in Claim 1, wherein said gloves of said first pair of inner gloves (100,300) have a thickness in a fingertip and palm area of said gloves in a range of about 0.14 mm to about 0.20 mm.

7. A surgical glove set as set forth in Claim 1, wherein said gloves of said second pair of outer gloves (102,300) have a thickness in a fingertip area of said gloves in a range of about 0.24 mm to about 0.30 mm and/or a thickness in a palm area of said gloves in a range of about 0.21 mm to about 0.27 mm.

8. A surgical glove set as set forth in Claim 1, wherein said gloves of said first pair of inner gloves (100,300) have a length in a range of about 280 mm to about 290mmm, and said gloves of said second pair of outer gloves (102,300) have a length in a range of about 295 mm to about 310 mm.

9. A surgical glove set as set forth in Claim 1, wherein said gloves of said first pair of inner gloves (100,300) have an elongation at a break in a range of about 800-960%, and a modulus at 500% in a range of about 2.5 MPa to about 3.5 MPa.

10. A surgical glove set as set forth in Claim 1, wherein at least one glove of said pair of inner gloves (100,300) is packaged having a cuff portion (308) thereof folded over such that said cuff portion extends in a direction toward a fingertip portion of said glove, and having a distal end portion (320) of said folded cuff portion folded back over such that the distal end portion extends in a direction away from said fingertip portion of said glove, whereby said folded cuff portion remains spaced apart from a palm area of said glove.

## Patentansprüche

1. Chirurgie-Handschuhset, das aufweist:
ein erstes Paar Innenhandschuhe (100, 300); und
ein zweites Paar Außenhandschuhe (102, 300), die über den Innenhandschuhen zu tragen sind;
**dadurch gekennzeichnet, dass**
das Handschuhset als doppeltes Chirurgie-Handschuhset abgepackt ist;
wobei das erste Paar und das zweite Paar Handschuhe durch ein Trennblatt (208) voneinander getrennt sind, wobei das erste Paar Handschuhe auf dem zweiten Paar Handschuhe aufliegt;
wobei das erste Paar und das zweite Paar Handschuhe und das Trennblatt in einer einzigen Packung abgepackt sind.

2. Chirurgie-Handschuhset nach Anspruch 1, wobei das erste Paar Innenhandschuhe (100, 300) und das zweite Paar Außenhandschuhe (102, 300) jeweils zwei handspezifische Handschuhe aufweisen.

3. Chirurgie-Handschuhset nach Anspruch 2, wobei die Handschuhe des zweiten Paars Außenhandschuhe (102, 300) nebeneinander auf einem äußeren sterilen Blatt (202) angeordnet sind, und wobei die Handschuhe des ersten Paars Innenhandschuhe (100, 300) nebeneinander so angeordnet sind, dass sie auf dem zweiten Paar Außenhandschuhe aufliegen, wobei das Trennblatt (208) zwischen dem ersten Paar und dem zweiten Paar Handschuhe angeordnet ist.

4. Chirurgie-Handschuhset nach Anspruch 3, das des Weiteren einen sterilen Verpackungsbeutel (210) aufweist, wobei das äußere sterile Blatt (202) entlang einer Linie gefaltet ist, die sich zwischen jedem Paar nebeneinander liegender Innen- und Außenhandschuhe (102, 300) erstreckt, wobei durch das äußere sterile Blatt die ersten und zweiten Paar Handschuhe zwischen umgefalteten Abschnitten des gefalteten Blatts angeordnet sind, und wobei das äußere sterile Blatt und die ersten und zweiten Paar Handschuhe in dem sterilen Verpackungsbeutel versiegelt sind.

5. Chirurgie-Handschuhset nach Anspruch 1, wobei die Handschuhe des zweiten Paars Außenhandschuhe (102, 300) größer sind, als die Handschuhe des ersten Paars Innenhandschuhe (100, 300), z.B. sind die Handschuhe des zweiten Paars Außenhandschuhe um ca. 3% bis ca. 4,5% größer, als die Handschuhe des Paars Innenhandschuhe.

6. Chirurgie-Handschuhset nach Anspruch 1, wobei die Handschuhe des ersten Paars Innenhandschuhe (100, 300) in dem Fingerspitzen- und Handflächen-Bereich der Handschuhe eine Stärke im Bereich von ca. 0,14 mm bis ca. 0,20 mm haben.

7. Chirurgie-Handschuhset nach Anspruch 1, wobei die Handschuhe des zweiten Paars Außenhandschuhe (102, 300) in dem Fingerspitzen-Bereich der Handschuhe eine Stärke im Bereich von ca. 0,24 mm bis ca. 0,30 mm und/oder in dem Handflächen-Bereich der Handschuhe eine Stärke in einem Bereich von ca. 0,21 mm bis ca. 0,27 mm haben.

8. Chirurgie-Handschuhset nach Anspruch 1, wobei die Handschuhe des ersten Paars Innenhandschuhe (100, 300) eine Länge in einem Bereich von ca. 280 mm bis ca. 290 mm haben, und wobei die Handschuhe des zweiten Paars Außenhandschuhe (102, 300) eine Länge in einem Bereich von ca. 295 mm bis ca. 310 mm haben.

9. Chirurgie-Handschuhset nach Anspruch 1, wobei die Handschuhe des ersten Paars Innenhandschuhe (100, 300) eine Reißdehnung in einem Bereich von ca. 800 - 960% und ein Zugmodul bei 500% in einem Bereich von ca. 2,5 MPa bis ca. 3,5 MPa haben.

10. Chirurgie-Handschuhset nach Anspruch 1, wobei wenigstens ein Handschuh des Paars Innenhandschuhe (100, 300) so verpackt wird, dass ein Bündchenabschnitt (308) davon umgefaltet wird, so dass sich der Bündchenabschnitt zu dem Fingerspitzenabschnitt des Handschuhs hin erstreckt, und so, dass ein distaler Endabschnitt (320) des gefalteten Bündchenabschnitts zurückgefaltet wird, so dass sich der distale Endabschnitt von dem Fingerspitzenabschnitt des Handschuhs weg erstreckt, wodurch der gefaltete Bündchenabschnitt von dem Handflächen-Bereich des Handschuhs beabstandet bleibt.

## Revendications

1. Jeu de gants chirurgicaux, comprenant :
une première paire de gants intérieurs (100, 300) ; et
une deuxième paire de gants extérieurs (102, 300) à porter sur lesdits gants intérieurs ; **caractérisé en ce que :**
le jeu de gants est conditionné comme un jeu de gants chirurgicaux double ;
ladite première paire et ladite deuxième paire de gants étant séparées par une feuille de séparation (208), ladite première paire de gants étant superposée sur ladite deuxième paire de gants ;
ladite première paire et ladite deuxième paire de gants et ladite feuille de séparation étant conditionnées dans un emballage unique.

2. Jeu de gants chirurgicaux selon la revendication 1, dans lequel ladite première paire de gants intérieurs (100, 300) et ladite deuxième paire de gants extérieurs (102, 300) comprennent chacune deux gants spécifiques à une main.

3. Jeu de gants chirurgicaux selon la revendication 2, dans lequel lesdits gants de ladite deuxième paire de gants extérieurs (102, 300) sont placés côte à côte sur une feuille de champ stérile externe (202) et lesdits gants de ladite première paire de gants intérieurs (100, 300) sont placés côte à côte superposés sur ladite deuxième paire de gants extérieurs, ladite feuille de séparation (208) étant disposée entre ladite première paire et ladite deuxième paire de gants.

4. Jeu de gants chirurgicaux selon la revendication 3, comprenant en outre une poche d'emballage stérile (210), dans lequel ladite feuille de champ stérile externe (202) est repliée le long d'une ligne s'étendant entre chaque paire de gants intérieurs et extérieurs côte à côte (102, 300), ladite feuille de champ stérile externe ayant ladite première et ladite deuxième paire de gants disposées entre les parties repliées de la feuille pliée et dans lequel ladite feuille de champ stérile externe et ladite première et ladite deuxième paire de gants étant scellées dans ladite poche d'emballage stérile.

5. Jeu de gants chirurgicaux selon la revendication 1, dans lequel lesdits gants de ladite deuxième paire de gants extérieurs (102, 300) sont plus grands que lesdits gants de ladite première paire de gants intérieurs (100, 300), par exemple, lesdits gants de ladite deuxième paire de gants extérieurs sont d'environ 3 % à environ 4,5 % plus grands que lesdits gants de ladite paire de gants intérieurs.

6. Jeu de gants chirurgicaux selon la revendication 1, dans lequel lesdits gants de ladite première paire de gants intérieurs (100, 300) ont une épaisseur de l'ordre d'environ 0,14 mm à environ 0,20 mm.

7. Jeu de gants chirurgicaux selon la revendication 1, dans lequel lesdits gants de ladite deuxième paire de gants extérieurs (102, 300) ont une épaisseur dans un doigt desdits gants de l'ordre d'environ 0,24 mm à environ 0,30 mm et/ou une épaisseur dans la région de la paume de l'ordre d'environ 0,21 mm à environ 0,27 mm.

8. Jeu de gants chirurgicaux selon la revendication 1, dans lequel lesdits gants de ladite première paire de gants intérieurs (100, 300) ont une longueur de l'ordre d'environ 280 mm à environ 290 mm, et lesdits gants de ladite deuxième paire de gants extérieurs (102, 300) ayant une longueur de l'ordre d'environ 295 mm à environ 310 mm.

9. Jeu de gants chirurgicaux selon la revendication 1, dans lequel lesdits gants de ladite première paire de gants intérieurs (100, 300) ont un allongement à la rupture de l'ordre d'environ 800 à 960 % et un module à 500% de l'ordre d'environ 2,5 MPa à environ 3,5 MPa.

10. Jeu de gants chirurgicaux selon la revendication 1, dans lequel au moins un gant de ladite paire de gants intérieurs (100, 300) est empaqueté en ayant une partie de manchette (308) de celui-ci repliée sur celui-ci de sorte que ladite partie de manchette s'étende dans une direction vers une partie de doigt dudit gant et en ayant une extrémité distale (320) de ladite partie de manchette repliée, qui est repliée sur celui-ci de sorte que la partie d'extrémité distale s'étende dans une direction distante de ladite partie de doigt dudit gant, dans lequel ladite partie de manchette repliée reste espacée d'une région de paume dudit gant.
